# FASCICULE DE BREVET EUROPEEN

(11) **EP 2 034 892 B1**
(45) Date de publication et mention de la délivrance du brevet: **30.12.2009**
(21) Numéro de dépôt: 07823515.7
(22) Date de dépôt: 08.06.2007
(51) Int. Cl.: A61B 5/103, A43B 7/00

(54) **PROCEDE ET DISPOSITIF DE DETECTION ET DE PREVENTION DES ULCERES PLANTAIRES**
VERFAHREN UND VORRICHTUNG ZUR ERFASSUNG UND VERHINDERUNG PLANTARER GESCHWÜRE
METHOD AND DEVICE FOR DETECTING AND PREVENTING PLANTAR ULCERS

(30) Priorité: 09.06.2006 FR 0652073
(43) Date de publication de la demande: 18.03.2009
(73) Titulaire: UNIVERSITE JOSEPH FOURIER, 38041 Grenoble Cédex 9 (FR)
(72) Inventeur: VUILLERME, Nicolas, F-73800 Francin (FR); NOURY, Norbert, 38000 Grenoble (FR); PAYAN, Yohan, 38580 Allevard (FR); DEMONGEOT, Jacques, 38360 Sassenage (FR)
(74) Mandataire: de Beaumont, Michel
(86) Numéro de dépôt international: PCT/FR2007/051399
(87) Numéro de publication internationale: WO 2008/006995

(56) Documents cités:
- WO-A-03/079882
- DE-A1- 4 308 945
- JP-A- 2001 238 967
- US-A- 5 642 096
- US-A- 5 929 332

## Description

### Domaine de l'invention

La présente invention concerne un dispositif de prévention des ulcères plantaires.

### Exposé de l'art antérieur

Les ulcères plantaires deviennent un problème de santé publique non négligeable. En particulier, on observe une augmentation des ulcères plantaires proportionnelle à l'augmentation croissante des personnes présentant un surpoids ou étant en état d'obésité. De plus, indépendamment de leur poids, des personnes atteintes de pathologies chroniques affectant la circulation, par exemple les diabétiques, sont également susceptibles de souffrir d'ulcères plantaires. On observe également l'apparition d'ulcères plantaires après un traumatisme ou/et une intervention chirurgicale affectant un des membres inférieurs. En effet, après un traumatisme ou une intervention, une personne peut avoir du mal à utiliser normalement son membre traumatisé ou opéré et prendre une mauvaise position propre à provoquer l'apparition d'ulcères. Par ailleurs, après un traumatisme ou une intervention, une personne peut avoir une réticence psychologique à faire porter normalement son poids du côté traumatisé ou opéré ce qui favorise l'apparition d'ulcères plantaires sur le pied se trouvant du côté non traumatisé ou non opéré.

L'apparition d'ulcères plantaires est irréversible. De plus, les ulcères sont difficiles voire impossibles à soigner et conduisent fréquemment à une réduction de la mobilité de la personne atteinte voire à une amputation du pied atteint. De tels effets ont un impact grave sur la personne touchée tant sur le plan physique que sur le plan psychologique.

On a proposé de procéder à une "adaptation" de la marché et de la posture des populations à risque afin de réduire la probabilité de l'apparition des ulcères. Une telle "adaptation" passe essentiellement par un apprentissage de marche adapté au risque de la personne, en salle de gymnastique. Toutefois, un tel apprentissage n'est pas définitif et impose un rafraîchissement, un contrôle fréquent. Cela constitue une contrainte d'autant plus grande que les salles adaptées sont souvent concentrées dans les grandes agglomérations, et incorporées dans des centres hospitaliers. Outre les contraintes imposées à la personne devant suivre l'apprentissage, cela constitue souvent une gêne pour les établissements concernés en raison du matériel et du personnel immobilisé et ce d'autant plus que le suivi de telles personnes qui ne relèvent pas à proprement de soins médicaux n'entre pas dans leurs fonctions normales.

De plus, un tel apprentissage est rarement personnalisé et s'appuie sur des études essentiellement statistiques. Il n'existe pas de procédé ni de dispositif de détection de risques d'apparition d'ulcères plantaires pour une personne donnée. L'apprentissage peut alors s'avérer en partie inefficace. Le document US-A-5 642 096 décrit un dispositif de détection d'un risque d'apparition d'ulcère plantaire selon le preambule de la revendication 3.

La présente invention vise à proposer un procédé et un dispositif de détection de risque d'apparition d'ulcères plantaires.

La présente invention vise à proposer un tel dispositif qui soit simple et susceptible d'être utilisé par une personne seule en dehors de tout encadrement et de toute installation.

La présente invention vise également à proposer un dispositif de prévention des ulcères permettant de compenser automatiquement un risque d'ulcère plantaire.

### Résumé de l'invention

Pour atteindre ces objets et d'autres, la présente invention prévoit un procédé de détection d'un risque d'apparition d'ulcère plantaire, comportant les étapes suivantes :
surveiller au moins la température et la pression d'au moins une sole plantaire ;
dès qu'un premier des paramètres, température ou pression, dépasse une valeur seuil, ouvrir une première fenêtre temporelle de surveillance du premier paramètre ;
si à la fin de la première fenêtre temporelle le premier paramètre dépasse toujours la valeur seuil, surveiller pendant une seconde fenêtre temporelle le deuxième des paramètres ; et
affecter un coefficient de risque à l'événement rencontré.

Selon un mode de réalisation de la présente invention, le taux d'oxygène dans le sang est surveillé en tant que paramètre supplémentaire à la température et à la pression.

La présente invention prévoit également un procédé de prévention des ulcères plantaires, consistant à détecter un risque d'apparition d'ulcère plantaire au moyen d'un procédé selon un des modes de réalisation précédents, et à fournir un signal à au moins un actionneur.

Selon un mode de réalisation de la présente invention, un actionneur est propre à délivrer un signal d'avertissement à la personne présentant le risque.

Selon un mode de réalisation de la présente invention, un actionneur est propre à provoquer un mouvement réflexe propre à compenser le risque d'ulcère plantaire.

La présente invention prévoit également un dispositif de détection d'un risque d'apparition d'ulcère plantaire, caractérisé en ce qu'il comporte :
une première matrice de capteurs de la température d'au moins une plante de pied ;
une deuxième matrice de capteurs de la pression de la plante ;
des compteurs ; et
une unité de traitement propre à détecter tout dépassement de valeurs seuils par la température ou la pression fournie par l'un quelconque des capteurs, à déclencher un premier compteur dès l'apparition d'un dépassement par un premier des paramètres température ou pression, à déclencher un deuxième compteur de surveillance de la deuxième quelconque du deuxième des paramètres pression ou température si le dépassement du premier paramètre se maintient pendant une première durée fixée par le premier compteur, et à calculer un coefficient de risque lorsque le deuxième compteur a fini de compter une deuxième durée.

Selon un mode de réalisation de la présente invention, le dispositif comporte en outre une matrice de capteurs propres à mesurer le taux d'oxygène dans le sang en divers points de la sole plantaire, l'unité de traitement étant propre à traiter les mesures de taux d'oxygène dans le sang en tant que paramètre supplémentaire à la température et à la pression.

Selon un mode de réalisation de la présente invention, le dispositif comporte un actionneur propre à fournir à l'utilisateur du dispositif un signal d'avertissement de risque d'ulcère plantaire.

Selon un mode de réalisation de la présente invention, l'actionneur propre à fournir à l'utilisateur du dispositif un signal d'avertissement est un dispositif d'électrostimulation lingual.

La présente invention prévoit également un dispositif de prévention des ulcères plantaires, caractérisé en ce qu'il comporte un dispositif de détection des ulcères plantaires selon l'un quelconque des modes de réalisation précédents, et au moins un actionneur propre à provoquer un mouvement réflexe propre à compenser le risque d'ulcère plantaire.

Selon un mode de réalisation de la présente invention, un actionneur propre à provoquer un mouvement réflexe est un actionneur électromusculaire.

### Brève description des dessins

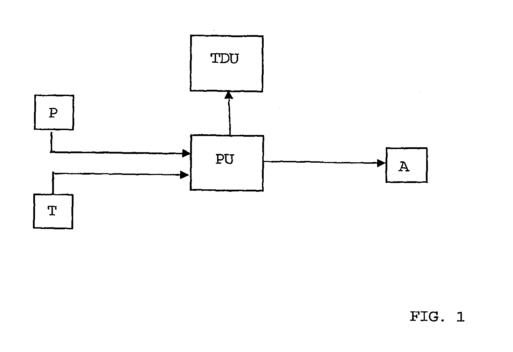

Ces objets, caractéristiques et avantages, ainsi que d'autres de la présente invention seront exposés en détail dans la description suivante de modes de réalisation particuliers faite à titre non-limitatif en relation avec la figure 1 qui illustre schématiquement un dispositif de prévention d'ulcères plantaires.

### Description détaillée

Selon un mode de réalisation de la présente invention, la chaussure d'une personne appartenant à une population à risque en termes d'apparition d'ulcères plantaires est équipée de divers capteurs.

En particulier, la chaussure comporte une matrice ou nappe P de capteurs de pression ayant la forme du pied et propre à détecter la pression de la sole plantaire. De préférence, la nappe P présente un resserrement du nombre de capteurs aux emplacements les plus exposés, notamment les têtes métatarsiennes, en particulier les première et cinquième, ainsi qu'au niveau du talon.

La chaussure comporte également une matrice T de capteurs de température propres à mesurer la température en divers points de la sole plantaire. De préférence, le nombre de capteurs de température est plus élevé au niveau des emplacements auxquels la probabilité d'apparition d'un ulcère est la plus élevée.

Les informations provenant des capteurs P et T sont transmises à une unité de traitement PU. L'unité PU détecte alors l'apparition d'une surpression ou d'une température trop élevée. Par exemple, l'unité PU compare individuellement les données de température prélevées en différentes zones à des valeurs seuils. Les valeurs seuils dépendent de la zone associée au capteur considéré. Si l'unité PU détecte un dépassement d'un seuil dans au moins une des zones, elle déclenche pour cette zone un compteur d'une première durée.

Si, avant la fin de la première durée, l'état de température trop élevée disparaît, l'unité PU ne tient pas compte de cette information.

Si, à la fin de la première durée, l'état de température trop élevée se maintient, l'unité PU mémorise cet événement, c'est-à-dire la zone et le degré de température trop élevée. De plus, l'unité PU provoque une détection d'une éventuelle surpression en comparant pendant une deuxième durée les données de pression provenant de la zone en température trop élevée à au moins un seuil.

Si la température trop élevée disparaît au cours de la deuxième durée et qu'aucune surpression n'est détectée, l'unité PU se borne à mémoriser les informations d'emplacement et de durée. La mémoire de l'événement est conservée pendant une troisième durée. Si aucun autre événement de température trop élevée n'affecte la zone considérée pendant la troisième durée, l'événement est oublié.

Si au cours de la deuxième durée la température trop élevée se maintient, l'unité PU affecte à cette zone un coefficient de risque d'apparition d'ulcères. Ce coefficient est modulé en fonction de l'apparition d'une surpression. Le coefficient est plus élevé si une pression plus élevée que la normale a été détectée, pendant la deuxième durée.

Une fois écoulée la deuxième durée, l'unité PU prend en compte l'historique mémorisé pour la zone concernée et modifie le cas échéant le coefficient de risque en fonction des événements passés survenus pendant une fenêtre de temps passée correspondant à la troisième durée et s'achevant à la fin de la deuxième durée.

Le coefficient de risque est ensuite comparé à au moins un seuil de prévention.

Une fois dépassé le seuil de prévention, l'unité PU envoie selon un mode de réalisation de la présente invention un signal d'avertissement à la personne.

Selon un mode de réalisation de la présente invention, le signal est envoyé vers un dispositif d'électrostimulation lingual TDU. Un tel dispositif TDU est constitué généralement au moins d'un palais artificiel propre à être placé dans la cavité buccale et portant une matrice d'électrodes susceptibles d'entrer en contact au moins avec la surface supérieure de la langue. Le signal présente alors un codage propre à activer des zones prédéfinies de la matrice d'électrodes. Le codage permet d'indiquer à la personne soit l'emplacement et le degré du risque, soit une action à prendre afin de réduire le risque d'apparition d'un ulcère plantaire. Le mode de codage est choisi en fonction de la personne affectée, de sa capacité à analyser la situation.

Selon un autre mode de réalisation de la présente invention, que l'information sur le risque d'apparition de l'ulcère soit ou non transmise à l'utilisateur, l'unité PU est propre à déclencher la mise en route d'actionneurs A placés dans la chaussure et propres à provoquer une disparition du risque d'ulcère.

De tels actionneurs A sont par exemple des actionneurs électromusculaires (electromyographic actuators) placés sous la sole plantaire au niveau des muscles mobilisateurs de la cheville et qui provoquent une stimulation de contraction d'un tendon, d'un récepteur tactile ou d'un muscle, correspondant à la zone présentant un risque. Cette stimulation est ressentie inconsciemment par la personne comme un mouvement réel de son propre corps et la conduit à effectuer par réflexe un déplacement de la zone propre à compenser ce mouvement, provoquant un déplacement propre à faire disparaître le risque.

Un autre type d'actionneurs A est un actionneur propre à effectuer une stimulation mécanique. Cette stimulation peut être perçue, comme la stimulation électromusculaire précédente, comme un déplacement du pied et provoquer un déplacement réflexe du pied propre à compenser le risque d'ulcères. De plus, une stimulation mécanique s'apparente à un massage et provoque des micro-déplacements qui soulagent la zone affectée.

On notera que les actionneurs peuvent également être placés non pas sous les pieds, mais sur d'autres parties du corps, notamment les membres inférieurs afin de provoquer une modification de la posture. En effet, un ulcère plantaire peut également résulter par exemple d'une mauvaise position de la jambe.

On a ainsi prévu un dispositif et un procédé de détection des risques d'ulcères plantaires.

De plus, complété par les actionneurs, le dispositif permet de prévenir la formation des ulcères.

Le procédé et le dispositif selon la présente invention présentent de nombreux avantages.

Notamment, ils permettent de dispenser la personne de fréquenter des salles spécialisées ou de réduire la fréquence des visites. Cela supprime les contraintes associées tant pour la personne équipée que pour l'établissement.

En outre, le dispositif est embarqué et discret : les semelles portant les capteurs et actionneurs appropriés peuvent être conçus pour s'insérer dans une chaussure, l'unité PU est un boîtier de dimension réduite qui peut être placé dans une poche ou fixé à une ceinture. De préférence, le dispositif est conçu avec une technologie de communication sans fil entre l'unité de traitement PU et les capteurs T et P et entre l'unité de traitement PU et les actionneurs TDU et A.

Bien entendu, la présente invention est susceptible de diverses variantes et modifications qui apparaîtront à l'homme de l'art. En particulier, on a décrit précédemment un mode de réalisation dans lequel la détection du risque d'ulcères est basée sur une première détection d'une température trop élevée qui provoque l'ouverture d'une fenêtre temporelle pendant laquelle l'unité PU détecte l'apparition d'une éventuelle surpression. Toutefois, il serait également possible d'effectuer une première détection d'une surpression puis de provoquer l'ouverture d'une fenêtre temporelle pendant laquelle l'unité PU détecte l'apparition d'une température trop élevée.

De préférence, selon un mode de réalisation de la présente invention, l'unité PU surveille en permanence la température et la pression et est susceptible de provoquer, dès que l'un de ces deux paramètres a dépassé une valeur seuil, l'ouverture d'une fenêtre temporelle pendant laquelle elle détecte l'apparition d'une éventuelle valeur excessive de l'autre paramètre.

On notera que les seuils de déclenchement des compteurs et les durées de surveillance varient en fonction de la zone concernée. Ainsi, lors d'une marche normale, le talon porte pendant une courte durée une masse importante et s'échauffe brutalement de façon correspondante pendant une brève durée correspondant au premier appui d'un pied levé. De façon similaire, lorsqu'un pied est en train d'être levé, la masse est momentanément totalement portée par les têtes métatarsiennes. Un niveau de pression trop élevée diffère de plus en fonction de la zone considérée. Ainsi un niveau considéré comme normal dans la zone du talon sera considéré comme excessif dans la zone d'une tête métatarsienne. Les niveaux de seuils programmés dans l'unité de traitement dépendent du poids, de la taille et de la morphologie de l'individu.

Selon un mode de réalisation de la présente invention, le coefficient de risque est modulé en fonction de la valeur du dépassement de la température et de la pression par rapport à la normale. Ainsi, pour une même température trop élevée, le coefficient sera plus faible dans le cas où la pression n'a dépassé la normale que vers la fin de la deuxième durée par rapport au cas où la pression était déjà supérieure à la normale au début de la deuxième durée.

Par ailleurs, dans les modes de réalisation décrits précédemment seules la température et la pression ont été prises en compte. On notera toutefois que d'autres paramètres peuvent être pris en compte. En particulier, pour une personne présentant un risque élevé d'apparition d'ulcères, on pourra utiliser un ou plusieurs capteurs propres à détecter le taux d'oxygène dans le sang. Un tel paramètre est utilisé pour accélérer la détection d'un risque. En effet, si une pression et/ou une température trop élevée sont détectées dans une zone donnée le risque d'ulcère est d'autant plus élevé que le taux d'oxygène dans le sang est plus faible.

## Revendications

1. Procédé de détection d'un risque d'apparition d'ulcère plantaire, comportant les étapes suivantes :
surveiller au moins la température et la pression d'au moins une sole plantaire ;
dès qu'un premier des paramètres, température ou pression, dépasse une valeur seuil, ouvrir une première fenêtre temporelle de surveillance du premier paramètre ;
si à la fin de la première fenêtre temporelle le premier paramètre dépasse toujours ladite valeur seuil, surveiller pendant une seconde fenêtre temporelle le deuxième des paramètres ; et
affecter un coefficient de risque à l'événement rencontré.

2. Procédé selon la revendication 1, dans lequel le taux d'oxygène dans le sang est surveillé en tant que paramètre supplémentaire à la température et à la pression.

3. Dispositif de détection d'un risque d'apparition d'ulcère plantaire, comportant :
une première matrice (T) de capteurs de la température d'au moins une plante de pied ;
une deuxième matrice (P) de capteurs de la pression de ladite plante ;
une unité de traitement (PU) propre à détecter tout dépassement de valeurs seuils par la température ou la pression fournie par l'un quelconque des capteurs, **caracterisé en ce que** le dispositif comprend des compteurs, l'unité de traitement est propre à déclencher un premier compteur dès l'apparition d'un dépassement par un premier des paramètres température ou pression, à déclencher un deuxième compteur de surveillance de la deuxième quelconque du deuxième des paramètres pression ou température si le dépassement du premier paramètre se maintient pendant une première durée fixée par le premier compteur, et à calculer un coefficient de risque lorsque le deuxième compteur a fini de compter une deuxième durée.

4. Dispositif selon la revendication 3, comportant en outre une matrice de capteurs propres à mesurer le taux d'oxygène dans le sang en divers points de la sole plantaire, l'unité de traitement étant propre à traiter les mesures de taux d'oxygène dans le sang en tant que paramètre supplémentaire à la température et à la pression.

5. Dispositif selon la revendication 3, **caractérisé en ce qu'**il comporte un actionneur propre à fournir à l'utilisateur du dispositif un signal d'avertissement de risque d'ulcère plantaire.

6. Dispositif selon la revendication 5, dans lequel l'actionneur propre à fournir à l'utilisateur du dispositif un signal d'avertissement est un dispositif d'électrostimulation lingual.

7. Dispositif de prévention des ulcères plantaires, **caractérisé en ce qu'**il comporte un dispositif de détection des ulcères plantaires selon l'une quelconque des revendications 3 à 6 et au moins un actionneur propre à provoquer un mouvement réflexe propre à compenser le risque d'ulcère plantaire.

8. Dispositif selon la revendication 7, dans lequel un actionneur propre à provoquer un mouvement réflexe est un actionneur électromusculaire.

## Claims

1. A method for detecting a risk of formation of a plantar ulcer, comprising the steps of:
monitoring at least the temperature and the pressure of at least one plantar sole;
as soon as a first one of the parameters, temperature or pressure, exceeds a threshold value, opening a first time window for monitoring the first parameter;
if, at the end of the first time window, the first parameter still exceeds the threshold value, monitoring during a second time window the second parameter; and
assigning a risk coefficient to the encountered event.

2. The method of claim 1, wherein the blood oxygen level is monitored as parameter additional to temperature and pressure.

3. A device for detecting a risk of formation of a plantar ulcer, comprising:
a first array (T) of sensors of the temperature of at least one foot sole;
a second array (P) of sensors of the sole pressure;
a processing unit (PU) capable of detecting any exceeding of threshold values by the temperature or the pressure provided by any of the sensors, **characterized in that** the device comprises counters, the processing unit being capable of starting a first counter as soon as a first of the temperature or pressure parameters has exceeded the threshold value, of starting a second counter for monitoring the second one of the pressure or temperature parameters if the exceeding of the first parameter is maintained for a first duration set by the first counter, and of calculating a risk coefficient when the second counter has finished counting for a second duration.

4. The device of claim 3, further comprising an array of sensors for measuring the blood oxygen level at various points of the plantar sole, the processing unit being capable of processing the blood oxygen level measurements as a parameter additional to the temperature and the pressure.

5. The device of claim 3, comprising an actuator capable of delivering to the user of the device a plantar ulcer risk warning signal.

6. The device of claim 5, wherein the actuator capable of delivering a warning signal to the user of the device is a device of electrical stimulation of the tongue.

7. A device for preventing plantar ulcers, comprising a device for detecting plantar ulcers according to any of claims 3 to 6, and at least one actuator capable of causing a reflex movement capable of compensating for the risk of plantar ulcer.

8. The device of claim 7, wherein an actuator capable of causing a reflex movement is an electromyographic actuator.

## Patentansprüche

1. Verfahren zum Detektieren eines Risikos für die Entwicklung eines plantaren Ulkus, welches folgende Schritte aufweist:
Überwachen von zumindest der Temperatur und dem Druck von mindestens einer Fußsohle;
sobald ein erster der Parameter, Temperatur oder Druck, einen Grenzwert überschreitet, Öffnen eines ersten Zeitfensters zum Überwachen des ersten Parameters;
wenn am Ende des ersten Zeitfensters der erste Parameter immer noch den Grenzwert überschreitet, Überwachen des zweiten Parameters während eines zweiten Zeitfensters; und
Zuweisen eines Risikokoeffizienten an das aufgetretene Ereignis.

2. Verfahren nach Anspruch 1, wobei der Sauerstoffgehalt des Blutes als Parameter zusätzlich zu Temperatur und Druck überwacht wird.

3. Vorrichtung zum Detektieren eines Risikos zur Entwicklung eines plantaren Ulkus, welche Folgendes aufweist:
eine erste Sensoren-Anordnung (T) für die Temperatur von mindestens einer Fußsole;
eine zweite Sensoren-Anordnung (P) für den Sohlendruck;
eine Verarbeitungseinheit (PU), welche in der Lage ist, jegliches Überschreiten der Grenzwerte durch die Temperatur oder den Druck, die von einem der Sensoren geliefert werden, zu detektieren, **dadurch gekennzeichnet, dass** die Vorrichtung Zähler aufweist, wobei die Verarbeitungseinheit in der Lage ist, einen ersten Zähler zu starten, sobald ein erster der Temperatur- oder Druckparameter den Grenzwert überschritten hat, einen zweiten Zähler zum Überwachen des zweiten der Druck- oder Temperaturparameter zu starten, wenn das Überschreiten des ersten Parameters für eine erste Dauer, die von dem ersten Zähler eingestellt ist, anhält, und einen Risikokoeffizienten zu berechnen, wenn der zweite Zähler das Zählen für eine zweite Zeitdauer beendet hat.

4. Vorrichtung nach Anspruch 3, welche ferner eine Sensorenanordnung zum Messen des Sauerstoffgehaltes des Blutes an unterschiedlichen Punkten der Fußsohle aufweist, wobei die Verarbeitungseinheit in der Lage ist, die Sauerstoffgehaltmessungen des Blutes als einen Parameter zusätzlich zu der Temperatur und dem Druck zu verarbeiten.

5. Vorrichtung nach Anspruch 3, welche einen Betätiger aufweist, der in der Lage ist, dem Benutzer der Vorrichtung ein Warnsignal für das Risiko eines plantaren Ulkus zu liefern.

6. Vorrichtung nach Anspruch 5, wobei der Betätiger, welcher in der Lage ist dem Benutzer der Vorrichtung ein Warnsignal zu liefern; eine Vorrichtung zur Elektrostimulation der Zunge ist.

7. Vorrichtung zum Verhindern plantarer Ulcera, welche eine Vorrichtung zum Detektieren plantarer Ulcera gemäß einem der Ansprüche 3 bis 6 und mindestens einen Betätiger aufweist, welcher in der Lage ist, eine Reflexbewegung zu bewirken, die in der Lage ist, das Risiko für ein plantares Ulkus zu kompensieren.

8. Vorrichtung nach Anspruch 7, wobei ein Betätiger, welcher in der Lage ist, eine Reflexbewegung zu bewirken, ein elektromyographischer Betätiger ist.
